# FASCICULE DE BREVET EUROPEEN

(11) **EP 4 607 166 B1**
(45) Date de publication et mention de la délivrance du brevet: **09.12.2026**
(21) Numéro de dépôt: 25305112.2
(22) Date de dépôt: 28.01.2025
(51) Int. Cl.: G01K 3/14, G01K 7/42, G01N 25/18, G01N 33/00, G01R 21/02, G01R 21/14, G01R 31/08, G01R 31/58

(54) **SYSTÈME DE DÉTERMINATION D'UNE QUANTITÉ D'ÉMISSIONS DE DIOXYDE DE CARBONE RÉSULTANT DE L'ÉCHAUFFEMENT D'UN CONDUCTEUR ÉLECTRIQUE D'UN CÂBLE ÉLECTRIQUE PAR EFFET JOULE**
SYSTEM ZUR BESTIMMUNG EINER MENGE VON KOHLENDIOXIDEMISSIONEN AUS DER ERWÄRMUNG EINES ELEKTRISCHEN LEITERS EINES ELEKTRISCHEN KABELS DURCH JOULE-EFFEKT
SYSTEM FOR DETERMINING AN AMOUNT OF CARBON DIOXIDE EMISSIONS RESULTING FROM THE HEATING OF AN ELECTRIC CONDUCTOR OF AN ELECTRIC CABLE BY JOULE EFFECT

(30) Priorité: 21.02.2024 FR 2401716
(43) Date de publication de la demande: 27.08.2025
(73) Titulaire: Nexans, 92800 Puteaux (FR)
(72) Inventeur: GUFFOND, Raphaël, 92400 COURBEVOIE (FR)
(74) Mandataire: Cabinet Boettcher

(56) Documents cités:
- WO-A1-2012/127601
- PSOMOPOULOS C.S. ET AL: "Electricity savings and CO2 emissions reduction in buildings sector: How important the network losses are in the calculation?", ENERGY, vol. 35, no. 1, 1 January 2010 (2010-01-01), AMSTERDAM, NL, pages 485 - 490, XP093200948, ISSN: 0360-5442, DOI: 10.1016/j.energy.2009.10.016
- VORSIC ZIGA ET AL: "New Method for Calculating the Heating of the Conductor", ENERGIES, vol. 12, no. 14, 18 July 2019 (2019-07-18), CH, pages 2769, XP093201169, ISSN: 1996-1073, DOI: 10.3390/en12142769

## Description

### Domaine technique

La présente invention concerne un système de détermination d'une quantité d'émissions de dioxyde de carbone résultant de l'échauffement d'un conducteur électrique d'un câble électrique par effet Joule.

Plus spécifiquement, l'invention concerne un système de détermination non-invasif.

### Arrière-plan technologique

Dans un réseau de distribution électrique, un échauffement du conducteur électrique d'un câble électrique se produit par effet Joule lors de la circulation d'un courant le long du conducteur électrique. Avec cette augmentation de température, la résistance du conducteur électrique augmente, ce qui entraîne une augmentation de la perte de puissance.

Cette perte de puissance annuelle a un impact direct sur la quantité des émissions de dioxyde de carbone (CO₂).

Il n'existe pas à ce jour de solutions intégrées ou rapportables de détermination de ces émissions de dioxyde de carbone résultant de l'échauffement d'un conducteur électrique d'un câble électrique par effet Joule.

Il existe donc un besoin pour un système de détermination d'une quantité d'émissions de dioxyde de carbone résultant de l'échauffement d'un conducteur électrique d'un câble électrique par effet Joule, directement intégré sur un câble électrique ou rapportable sur celui-ci.

### Résumé de l'invention

Pour cela, l'invention propose un système de détermination d'une quantité d'émissions de dioxyde de carbone résultant de l'échauffement d'un conducteur électrique d'un câble électrique par effet Joule, ledit système de détermination comprenant :
- un câble électrique comprenant au moins un conducteur électrique et au moins une couche de matériau entourant ledit au moins un conducteur,
- une unité de mesure associée au câble électrique, ladite unité de mesure comprenant au moins un capteur de température et un dispositif de mesure de l'intensité électrique I_{cond} d'un courant électrique circulant dans le conducteur électrique,
- une unité de calcul configurée pour communiquer des informations avec l'unité de mesure, l'unité de calcul étant configurée pour déterminer la température de conducteur Θ_{cond} au moyen dudit au moins un capteur de température, ladite unité de calcul étant en outre configurée pour déterminer une quantité d'émissions de dioxyde de carbone résultant de l'échauffement du conducteur électrique par effet joule en fonction de la température de conducteur Θ_{cond} et de l'intensité électrique I_{cond} dans le conducteur électrique.

L'intégration d'une unité de mesure et d'une unité de calcul configurée pour déterminer la température de conducteur Θ_{cond} et l'intensité électrique I_{cond} au sein d'un même système de détermination rend possible la détermination de la quantité des émissions de CO₂ induites par l'échauffement du conducteur électrique.

Selon un mode de réalisation du système de détermination, l'unité de calcul est configurée pour déterminer une résistance électrique R_{c} du conducteur électrique en fonction de la température de conducteur Θ_{cond}.

Cette résistance électrique R_{c} est notamment déterminée comme suit : ${R}_{c} = {R}_{0} \times \left(1+{α}_{20}\times \left({Θ}_{cond}-20\right)\right) \times \left(1+{y}_{s}+{y}_{p}\right)$

Selon un mode de réalisation du système de détermination, l'unité de calcul est configurée pour déterminer une perte de puissance PₒL en fonction de l'intensité électrique I_{cond} dans le conducteur électrique et de la résistance électrique R_{c} du conducteur électrique, l'unité de calcul étant configurée pour déterminer la quantité d'émissions de dioxyde de carbone en fonction de ladite perte de puissance PₒL.

Cette perte de puissance PₒL est notamment déterminée comme suit : $PoL = Rc \times {I}_{cond}^{2}$

Selon un mode de réalisation du système de détermination, celui-ci comprend en outre un boîtier de mesure monté sur le câble électrique, ledit boîtier de mesure comprenant au moins l'un parmi ledit au moins un capteur de température et le dispositif de mesure de l'intensité électrique I_{cond}.

Selon un mode de réalisation du système de détermination, le boîtier de mesure comprend en outre l'unité de calcul.

Selon un mode de réalisation du système de détermination, le boîtier de mesure configuré pour être monté de manière amovible sur le câble électrique.

Selon un mode de réalisation du système de détermination, le boîtier de mesure comprend un dispositif de fixation au câble électrique.

Selon un mode de réalisation du système de détermination, ledit au moins un capteur de température est disposé sur une surface externe de ladite au moins une couche de matériau pour mesurer une température périphérique Θ_{b1} au niveau de la surface externe de ladite au moins une couche de matériau, l'unité de calcul étant configurée pour déterminer la température de conducteur Θ_{cond} en fonction de la température périphérique Θ_{b1}.

L'utilisation d'un ou plusieurs capteurs de température externes au câble électrique permet une détermination de la température de conducteur Θ_{cond} non invasive et non-destructrice.

Selon un mode de réalisation du système de détermination, celui-ci comprend en outre :
- une couche additionnelle de matériau disposée autour de ladite au moins une couche de matériau et recouvrant ledit au moins un capteur de température,
- au moins un capteur de température additionnel disposé sur une surface externe de ladite couche de matériau additionnelle pour mesurer une température périphérique additionnelle Θ_{b2} au niveau de la surface externe de ladite au moins une couche additionnelle de matériau.

Selon un mode de réalisation du système de détermination, l'unité de calcul est configurée pour déterminer la température de conducteur Θ_{cond} en fonction de la température périphérique Θ_{b1} et de la température périphérique additionnelle Θ_{b2}.

Ladite au moins une couche de matériau du câble électrique présente une résistance thermique de couche T₁.

L'unité de calcul peut comprendre une unité de détermination de la température de conducteur Θ_{cond}. Ainsi, l'unité de détermination est configurée pour déterminer la température de conducteur Θ_{cond} en fonction de la température périphérique Θ_{b1} mesurée, la résistance thermique de couche T₁ et le flux thermique W_{c} généré par la circulation d'un courant électrique dans le conducteur électrique.

La détermination de la température de conducteur Θ_{cond} se fait ici au moyen d'un modèle physique utilisant la température périphérique Θ_{b1} mesurée et la résistance thermique de couche T₁.

L'utilisation de ce modèle physique permet de s'affranchir de l'utilisation d'une température interne au câble électrique, i.e. mesurée via un composant disposé à proximité du conducteur, ou plus généralement à l'intérieur de la gaine externe du câble électrique.

Le modèle physique utilisé permet d'estimer la température de conducteur Θ_{cond} au moyen de la température périphérique Θ_{b1} mesurée et la résistance thermique de couche T₁.

Selon un mode de réalisation du système de détermination, celui-ci comprend en outre :
- au moins une couche additionnelle de matériau disposée autour de ladite au moins une couche de matériau et recouvrant ledit au moins un capteur de température, ladite couche additionnelle de matériau présentant une résistance thermique additionnelle T_{b},
- au moins un capteur de température additionnel disposé sur une surface externe de ladite couche de matériau additionnelle pour mesurer une température périphérique additionnelle Θ_{b2} au niveau de la surface externe de ladite au moins une couche additionnelle de matériau.

Selon un mode de réalisation du système de détermination, ladite couche additionnelle de matériau s'étend autour de ladite au moins une couche de matériau uniquement sur une portion de la longueur du câble électrique.

Selon un mode de réalisation du système de détermination, ladite au moins une couche additionnelle de matériau comprend :
- une première portion de couche additionnelle présentant une première résistance thermique additionnelle T_{b}, et
- une deuxième portion de couche additionnelle présentant une deuxième résistance thermique additionnelle T'_{b}, les première T_{b} et deuxième T'_{b} résistances thermiques additionnelles étant différentes,
et dans lequel ledit au moins un capteur de température additionnel comprend :
- au moins un premier capteur de température additionnel disposé sur une surface externe de la première portion de couche additionnelle pour mesurer une première température périphérique additionnelle Θ_{b2},
- au moins un deuxième capteur de température additionnel disposé sur une surface externe de la deuxième portion de couche additionnelle pour mesurer une deuxième température périphérique additionnelle Θ'_{b2},
l'unité de détermination étant configurée pour déterminer la température de conducteur Θ_{cond} en outre en fonction de la première T_{b} et de la deuxième T'_{b} résistances thermiques additionnelles ainsi que de la première Θ_{b2} et de la deuxième Θ'_{b2} températures périphériques additionnelles.

Selon un mode de réalisation du système de détermination, lesdites au moins une première et au moins une deuxième couches additionnelles sont disposées sur des secteurs angulaires différents autour du conducteur électrique.

Lesdites au moins une première et au moins une deuxième couches additionnelles peuvent se recouvrir au moins partiellement.

Selon un mode de réalisation du système de détermination, lesdites au moins une première et au moins une deuxième couches additionnelles forment ensemble une couche s'étendant de manière continue autour du conducteur électrique dans un plan perpendiculaire à l'axe longitudinal d'extension du conducteur électrique.

Selon un mode de réalisation du système de détermination, ledit au moins un capteur de température comprend :
- au moins un premier capteur disposé sur la surface externe de ladite au moins une couche de matériau, entre ladite surface externe et la première portion de couche additionnelle, pour mesurer une température périphérique Θ_{b1} au niveau de la surface externe de ladite au moins une couche de matériau,
- au moins un deuxième capteur disposé sur la surface externe de ladite au moins une couche de matériau, entre ladite surface externe et la deuxième portion de couche additionnelle, pour mesurer une température périphérique Θ'_{b1} au niveau de la surface externe de ladite au moins une couche de matériau.

Selon un mode de réalisation du système de détermination, l'unité de détermination est configurée pour déterminer la température de conducteur Θ_{cond} sur la base de l'équation suivante : ${θ}_{conducteur} = \frac{\left({θ}_{b 1}{T}_{b}-θ{′}_{b 1}T{′}_{b}\right) \times \left(\left({θ}_{b 1}-{θ}_{b 2}\right)\right)}{{T}_{b} \left(θ{′}_{b 1}-θ{′}_{b 2}\right) - {T}_{b}^{′} \left({θ}_{b 1}-{θ}_{b 2}\right)}$
T₁ étant la résistance thermique de ladite au moins une couche de matériau,
T_{b} étant la première résistance thermique additionnelle de la première couche additionnelle de matériau,
T'_{b} étant la deuxième résistance thermique additionnelle de la deuxième couche additionnelle de matériau,
Θ_{b1} est la température périphérique mesurée par ledit au moins un premier capteur de température,
Θ_{b2} est la température périphérique additionnelle mesurée par ledit au moins un premier capteur de température additionnel,
Θ'_{b1} est la température périphérique mesurée par ledit au moins un deuxième capteur de température,
Θ'_{b2} est la température périphérique additionnelle mesurée par ledit au moins un deuxième capteur de température additionnel.

Selon un mode de réalisation du système de détermination, les première et deuxième portions de couche additionnelle sont réalisées dans un matériau différent.

Selon un mode de réalisation du système de détermination, les première et deuxième portions de couche additionnelle présentent une épaisseur différente, prise perpendiculairement à un axe longitudinal d'extension du câble électrique.

Selon un mode de réalisation du système de détermination, ladite au moins une couche de matériau comprend une gaine externe formant ladite surface externe, ledit au moins un capteur de température étant disposé sur ladite gaine externe.

Selon un mode de réalisation du système de détermination, l'unité de détermination est configurée pour déterminer une température de conducteur Θ_{cond} en fonction de la température périphérique Θ_{b1} mesurée et la résistance thermique de couche T₁ et le flux thermique W_{c} généré par la circulation d'un courant électrique dans le conducteur électrique.

Selon un mode de réalisation du système de détermination, l'unité de détermination est configurée pour déterminer la température de conducteur Θ_{cond} sur la base de l'équation suivante : ${Θ}_{cond} = {Θ}_{b 1} + {W}_{c} {* T}_{1}$

L'unité de détermination peut être disposée à proximité du câble électrique ou à distance de celui-ci.

Selon un mode de réalisation du système de détermination, ladite au moins une couche de matériau comprend une gaine externe formant ladite surface externe, ledit au moins un capteur de température étant disposé sur ladite gaine externe.

Selon un mode de réalisation du système de détermination, celui-ci comprend en outre un dispositif de mesure de l'intensité électrique I_{cond} d'un courant électrique circulant dans ledit au moins un conducteur électrique, l'unité de détermination étant configurée pour déterminer ladite température de conducteur Θ_{cond} en outre en fonction de cette intensité électrique I_{cond}.

Selon un mode de réalisation du système de détermination, le dispositif de mesure est un dispositif non-invasif, notamment du type d'un enroulement de Rogowski.

Selon un mode de réalisation du système de détermination, la température du conducteur Ocond est déterminée comme suit : ${Θ}_{cond} = {Θ}_{b 1} + {R}_{c} {{* I}_{cond}}^{2} {* T}_{1}$ R_{c} étant la résistance électrique du conducteur

Selon un mode de réalisation du système de détermination, celui-ci comprenant en outre :
- une couche additionnelle de matériau disposée autour de ladite au moins une couche de matériau et recouvrant ledit au moins un capteur de température, ladite couche additionnelle de matériau présentant une résistance thermique additionnelle Tb
- au moins un capteur de température additionnel disposé sur une surface externe de ladite couche de matériau additionnelle pour mesurer une température périphérique additionnelle Θb2 au niveau de la surface externe de ladite au moins une couche additionnelle de matériau.

Selon un mode de réalisation du système de détermination, ladite couche additionnelle de matériau s'étend autour de ladite au moins une couche de matériau uniquement sur une portion de la longueur du câble électrique.

Selon un mode de réalisation du système de détermination, l'unité de détermination est configurée pour déterminer la température de conducteur Θ_{cond} sur la base de l'équation suivante : ${θ}_{cond} = {θ}_{b 1} + \frac{\left({θ}_{b 1}-{θ}_{b 2}\right)}{{T}_{b}} \times {T}_{1}$
Θ_{b1} est la température périphérique mesurée par ledit au moins un capteur de température,
Θ_{b2} est la température périphérique additionnelle mesurée par ledit au moins un capteur de température additionnel,
T_{b} étant la résistance thermique additionnelle de la couche additionnelle de matériau,
T₁ étant la résistance thermique de ladite au moins une couche de matériau.

Selon un mode de réalisation du système de détermination, l'unité de détermination est configurée pour déterminer la température de conducteur Θ_{cond} en outre en fonction d'un échauffement W_{d} provenant d'une perte diélectrique dans ladite au moins une couche de matériau.

Selon un mode de réalisation du système de détermination, l'unité de détermination est configurée pour déterminer la température de conducteur Θ_{cond} sur la base de l'équation suivante : ${θ}_{cond} = {θ}_{b 1} + {T}_{1} \times \left({W}_{c}-\frac{{W}_{d}}{2}\right)$ W_{d} étant l'échauffement provenant d'une perte diélectrique dans ladite au moins une couche de matériau.

Selon un mode de réalisation du système de détermination, l'unité de détermination est configurée pour déterminer la température de conducteur Θ_{cond} sur la base de l'équation suivante : ${θ}_{cond} = {θ}_{b 1} + {T}_{1} \times \left(\frac{{θ}_{b 1} - {θ}_{b 2}}{{T}_{b}}-\frac{{W}_{d}}{2}\right)$ W_{d} étant l'échauffement provenant d'une perte diélectrique dans ladite au moins une couche de matériau.

Selon un mode de réalisation du système de détermination, ladite au moins une couche additionnelle de matériau et ledit au moins un capteur de température additionnel sont portés par le boîtier de mesure.

Ce boîtier de mesure est de préférence amovible par rapport au câble électrique de manière à réaliser une mesure ponctuelle et localisée sur le câble électrique. Ce boîtier est par exemple un accessoire de mesure.

Le boîtier de mesure présente de préférence une dimension suivant l'axe longitudinal du câble électrique de sorte qu'il s'étend uniquement sur une portion de la longueur du câble électrique.

### Brève description des figures

La description qui va suivre en regard des dessins annexés, donnés à titre d'exemples non limitatifs, fera bien comprendre en quoi consiste l'invention et comment elle peut être réalisée. Sur les figures annexées :
[fig. 1] La figure 1, représente une vue en perspective d'un système de détermination comprenant un câble électrique, une unité de mesure, une unité de calcul pour déterminer une quantité d'émissions de dioxyde de carbone résultant de l'échauffement d'un conducteur électrique du câble électrique;
[fig. 2] La figure 2 représente une vue en perspective d'un mode de réalisation du système de détermination de la figure 1 comprenant un boîtier de mesure monté sur le câble électrique, le boîtier de mesure comprend notamment l'unité de mesure;
[fig. 3] La figure 3 représente une vue en coupe du câble électrique selon une première configuration comprenant un conducteur électrique et au moins une couche de matériau;
[fig. 4] La figure 4 représente une vue en coupe du câble électrique de la figure 3 comprenant une pluralité de capteurs sur une surface externe de ladite au moins une couche de matériau;
[fig. 5] La figure 5 représente un schéma d'une première modélisation du câble électrique, selon un premier mode de détermination comprenant une détermination de l'intensité électrique du courant électrique circulant dans le conducteur électrique;
[fig. 6] La figure 6 représente une vue en coupe du câble électrique de la figure 3 selon un deuxième mode de détermination dans lequel le système de détermination comprend une couche additionnelle de matériau autour du câble électrique et une pluralité de capteurs additionnels disposés sur une surface externe de cette couche additionnelle de matériau;
[fig. 7] La figure 7 représente un schéma d'une deuxième modélisation du câble électrique selon le deuxième mode de détermination;
[fig. 8] La figure 8 représente une troisième modélisation du câble électrique dans laquelle les pertes diélectriques dans ladite au moins une couche de matériau sont prises en compte, selon le premier mode de détermination;
[fig. 9] La figure 9 représente une quatrième modélisation du câble électrique dans laquelle les pertes diélectriques dans ladite au moins une couche de matériau sont prises en compte, selon le deuxième mode de détermination;
[fig. 10] La figure 10 représente une vue en coupe du câble électrique selon une deuxième configuration comprenant un conducteur électrique, une ou plusieurs couches de matériau entourant le conducteur électrique et un écran entourant lesdites couches de matériau;
[fig. 11] La figure 11 une cinquième modélisation du câble électrique dans laquelle les pertes diélectriques dans ladite au moins une couche de matériau sont prises en compte ainsi que les pertes diélectriques dans l'écran;
[fig. 12] La figure 12 une sixième modélisation du câble électrique pour déterminer la température de conducteur Θ_{cond} de manière indépendante du milieu environnant;
[fig. 13] La figure 13 représente une vue en coupe du câble électrique de la figure 6 selon un deuxième mode de détermination dans lequel le système de détermination comprend une couche additionnelle de matériau formée d'une première et d'une deuxième portions de couche additionnelle et une pluralité de capteurs additionnels disposés sur une surface externe de chacune des première et deuxième portions de couche additionnelle de matériau;
[fig. 14] La figure 14 représente un schéma d'une septième modélisation du câble électrique utilisant la première portion de couche additionnelle de la figure 13;
[fig. 15] La figure 15 représente un schéma de la septième modélisation du câble électrique utilisant la deuxième portion de couche additionnelle de la figure 13;
[fig. 16] La figure 16 représente une équation de détermination de la température de conducteur selon la septième modélisation;
[fig. 17] La figure 17 représente un schéma d'une huitième modélisation du câble électrique utilisant la première portion de couche additionnelle de la figure 13;
[fig. 18] La figure 18 représente un schéma de la huitième modélisation du câble électrique utilisant la deuxième portion de couche additionnelle de la figure 13;
[fig. 19] La figure 19 représente une équation de détermination de la température de conducteur selon la huitième modélisation.

### Description de mode(s) de réalisation

Par souci de clarté, les mêmes références désignant les mêmes éléments selon l'état de l'art et selon l'invention sont utilisées pour toutes les figures.

Le concept de l'invention est décrit plus complètement ci-après avec référence aux dessins joints, sur lesquels des modes de réalisation du concept de l'invention sont montrés. Sur les dessins, la taille et les tailles relatives des éléments peuvent être exagérées à des fins de clarté. Des numéros similaires font référence à des éléments similaires sur tous les dessins. Cependant, ce concept de l'invention peut être mis en œuvre sous de nombreuses formes différentes et ne devrait pas être interprété comme étant limité aux modes de réalisation exposés ici. Au lieu de cela, ces modes de réalisation sont proposés de sorte que cette description soit complète, et communiquent l'étendue du concept de l'invention aux hommes du métier.

Une référence dans toute la spécification à « un mode de réalisation » signifie qu'une fonctionnalité, une structure, ou une caractéristique particulière décrite en relation avec un mode de réalisation est incluse dans au moins un mode de réalisation de la présente invention. Ainsi, l'apparition de l'expression « dans un mode de réalisation » à divers emplacements dans toute la spécification ne fait pas nécessairement référence au même mode de réalisation. En outre, les fonctionnalités, les structures, ou les caractéristiques particulières peuvent être combinées de n'importe quelle manière appropriée dans un ou plusieurs modes de réalisation. De plus, le terme « comprenant » n'exclut pas d'autres éléments ou étapes.

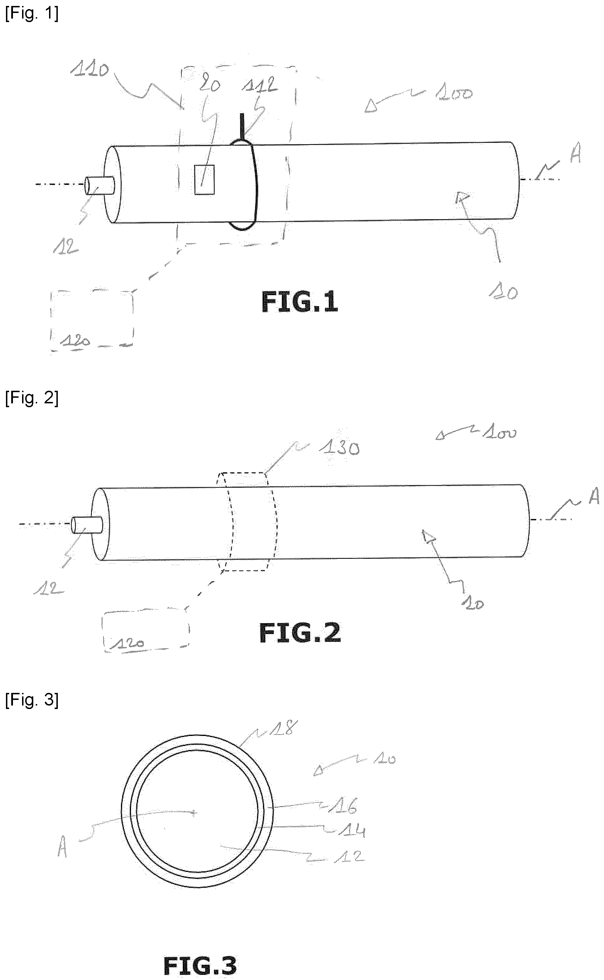

Un système de détermination 100 d'une quantité d'émissions de dioxyde de carbone résultant de l'échauffement d'un conducteur électrique d'un câble électrique par effet Joule est illustré sur la figure 1.

Ce système de détermination 100 comprend un câble électrique 10 comprenant au moins un conducteur électrique 12 et au moins une couche de matériau entourant ledit au moins un conducteur. Cette couche de matériau est par exemple une couche isolante.

Le câble électrique 10 s'étend le long d'un axe longitudinal A.

Ce système de détermination 100 comprend une unité de mesure 110 associée au câble électrique 10. L'unité de mesure comprend au moins un capteur de température 20 et un dispositif de mesure 112 de l'intensité électrique I_{cond} d'un courant électrique circulant dans le conducteur électrique 12.

Le dispositif de mesure 112 est de préférence un dispositif non-invasif, notamment du type d'un enroulement de Rogowski.

Le système de détermination 100 comprend en outre une unité de calcul 120 configurée pour communiquer des informations avec l'unité de mesure 110.

L'unité de calcul 120 est configurée pour déterminer la température de conducteur Θ_{cond} au moyen dudit au moins un capteur de température.

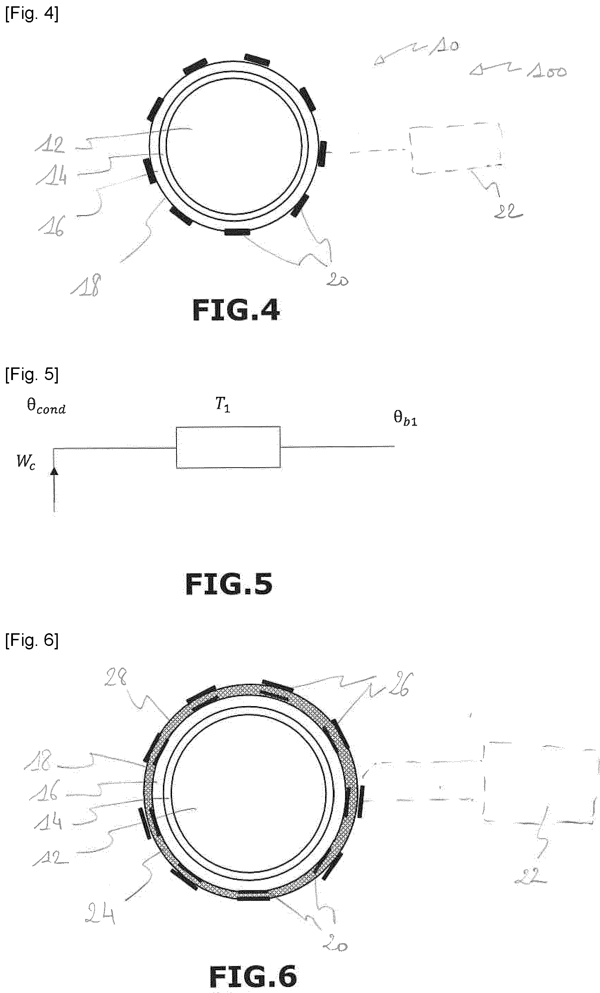

De manière préférée, ledit au moins un capteur de température 20 est disposé à l'extérieur du câble électrique, i.e. sur une surface externe de ce câble électrique 10. Dans cette configuration, la température de conducteur Θ_{cond} est déterminée au moyen d'un modèle physique décrit ci-après en lien avec les figures 4 à 12.

Dans une variante compatible avec l'invention, ledit au moins un capteur de température peut être disposé à l'intérieur du câble électrique 10. Dans cette variante, la température de conducteur Θ_{cond} est mesurée directement à proximité du conducteur électrique 12.

L'unité de calcul 120 est en outre configurée pour déterminer une quantité d'émissions de dioxyde de carbone résultant de l'échauffement du conducteur électrique par effet joule en fonction de la température de conducteur Θ_{cond} et de l'intensité électrique I_{cond} dans le conducteur électrique.

En référence à la figure 2, le système de détermination 100 peut comprendre un boîtier de mesure 130 dans lequel sont logés l'un ou plusieurs parmi ledit au moins un capteur de température 20 et le dispositif de mesure 112.

L'unité de mesure 110 entière est de préférence portée par le boîtier de mesure 130.

L'unité de calcul 120 est configurée pour être en communication avec l'unité de mesure 110. L'unité de calcul 120 peut être dissociée du boîtier de mesure 130 tel qu'illustré en figure 2 ou bien intégré à ce boîtier de mesure 130.

Ce boîtier de mesure 130 est par exemple monté de manière amovible par rapport au câble électrique 10 de manière à réaliser une mesure ponctuelle et localisée sur le câble électrique 10. Ce boîtier de mesure 130 est par exemple un accessoire de mesure portable.

Le boîtier de mesure 130 présente une dimension suivant l'axe longitudinal A de sorte qu'il s'étend uniquement sur une portion de la longueur du câble électrique 10. Le boîtier de mesure 130 s'étend de préférence autour du câble électrique 10, i.e. autour de l'axe longitudinal A.

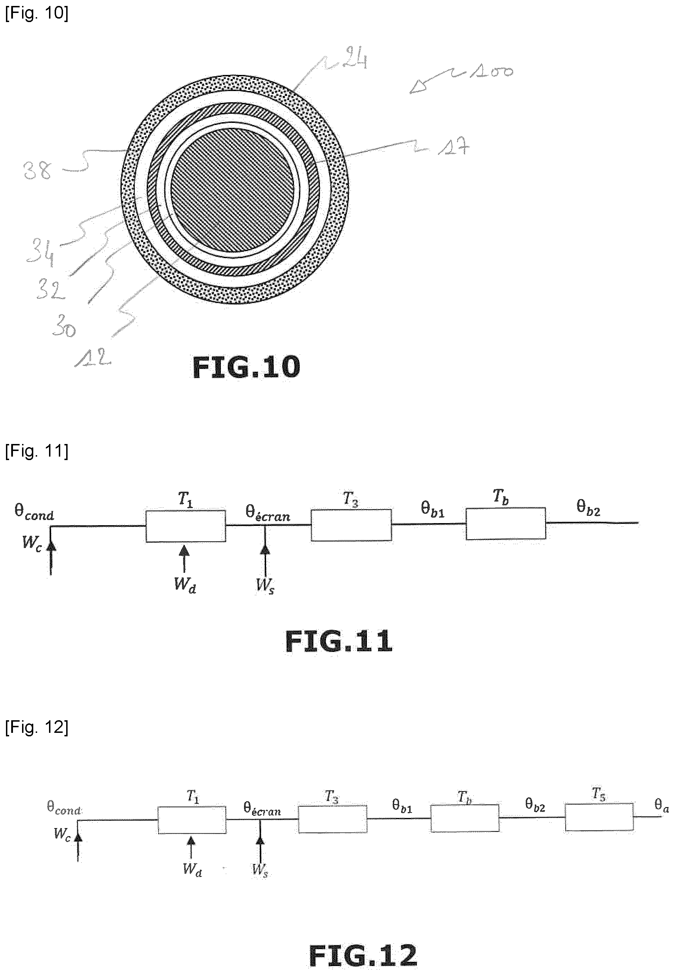

Le boîtier de mesure 130 peut également comprendre une structure additionnelle décrite ci-après, notamment en lien avec les figures 6 et 10.

Pour la détermination des émissions de CO₂, l'unité de calcul 120 est configurée pour déterminer une résistance électrique R_{c} du conducteur électrique en fonction de la température de conducteur Θ_{cond}.

Cette résistance électrique R_{c} est notamment déterminée comme suit : ${R}_{c} = {R}_{0} \times \left(1+{α}_{20}\times \left({Θ}_{cond}-20\right)\right) \times \left(1+{y}_{s}+{y}_{p}\right)$ avec
R₀ étant la résistance en courant continu du conducteur à 20°C, en Ohm,
Yₛ étant le facteur d'effet de peau, sans unité,
Yₚ étant le facteur d'effet de proximité, sans unité,
α₂₀ étant le coefficient de résistivité électrique, en K⁻¹ (i.e. par kelvin).

L'unité de calcul 120 est ensuite configurée pour déterminer une perte de puissance PₒL en fonction de l'intensité électrique I_{cond} mesurée dans le conducteur électrique et de la résistance électrique R_{c} du conducteur électrique déterminée.

Cette perte de puissance PₒL est notamment déterminée comme suit : $PoL = Rc \times {I}_{cond}^{2}$

L'unité de calcul 120 est configurée pour ensuite déterminer les pertes de puissance annuelles induites par l'échauffement du conducteur électrique 10. La quantité d'émissions de dioxyde de carbone est ensuite déterminée en fonction de ces pertes de puissance annuelles.

Pour déterminer la quantité d'émissions de CO₂ en fonction de ces pertes de puissance annuelles, il est possible d'utiliser un facteur d'échelle multiplié par les pertes de puissance annuelles.

D'une manière générale, la quantité d'émissions de CO₂ en fonction de ces pertes de puissance annuelles peut être déterminée en utilisant une relation définie par un opérateur de distribution (appelé « Distribution System Operator » en anglais).

En référence à la figure 3, un câble électrique 10 comprend un conducteur électrique 12, une première couche de matériau 14 autour du conducteur électrique 12 et une deuxième couche de matériau 16 autour de la première couche de matériau 14.

Le conducteur électrique 12 s'étend le long d'une axe longitudinal A.

Les première 14 et deuxième 16 couches de matériau s'étendent le long de l'axe longitudinal A, autour du conducteur électrique 12.

La première couche de matériau 14 est par exemple une couche formée d'un matériau isolant électriquement. La première couche de matériau 14 peut donc être considérée comme une couche isolante.

La deuxième couche de matériau 16 forme ici une couche externe du câble électrique 10. La deuxième couche de matériau 16 forme une surface externe 18 du câble électrique 10.

La deuxième couche de matériau 16 est par exemple une gaine externe.

De manière plus générale, le câble électrique 10 peut comporter une ou plusieurs couches de matériau entourant le conducteur électrique 12. Le câble électrique 10 peut notamment comprendre l'un ou plusieurs parmi : un écran, une couche semi-conductrice, une couche isolante, une gaine externe.

Dans une configuration préférée, le câble électrique 10 comprend autour du conducteur, par ordre de disposition du centre vers la périphérie : une couche semi-conductrice, une couche isolante, un écran et une gaine externe. Cette configuration correspond par exemple à un câble électrique configuré pour un réseau de moyenne tension (entre 1 et 52 kV).

Selon un mode de réalisation, le système de détermination 100 est de préférence non-invasif et/ou non destructif. En d'autres termes, ni ledit au moins un capteur de température 20 ni le dispositif de mesure 112 ne sont disposés à l'intérieur du câble électrique.

Dans ce mode de réalisation, l'unité de calcul 120 comprend une unité de détermination 22 configurée pour déterminer la température de conducteur Θ_{cond} au moyen d'un modèle physique utilisant notamment une température périphérique Θ_{b1} mesurée par ledit au moins un capter de température 20 disposé sur une surface externe du câble électrique 10.

La détermination de cette température de conducteur Θ_{cond} au moyen de ce modèle physique est décrite ci-après en lien avec les figures 4 à 12.

Tel qu'illustré sur la figure 4, le système de détermination 100 comprend ledit au moins un capteur de température 20 et une unité de détermination 22.

L'unité de détermination 22 appartient à l'unité de calcul 120.

Le système de détermination 100 peut comprendre une pluralité de capteurs de température 20 répartis autour de l'axe longitudinal A dans un même plan transversal à cet axe longitudinal A. Dans l'exemple de la figure 4, le système de détermination 100 comprend 9 capteurs de température 20.

Le ou les capteurs de température 20 sont configurés pour mesurer une température périphérique Θ_{b1}. Dans cette configuration où les capteurs de température 20 sont disposés au niveau de la surface externe 18 du câble électrique, la température périphérique Θ_{b1} correspond à la température en surface du câble électrique 10.

Le ou les capteurs de température 20 sont connectés à l'unité de détermination 22 de manière à communiquer la température périphérique Θ_{b1} à cette unité de détermination 22.

De préférence, les capteurs de température 20 sont équirépartis autour de l'axe longitudinal A. Le système de détermination 100 peut prévoir un ou plusieurs capteurs de température 20 répartis en outre le long de l'axe longitudinal A de manière à mesurer la température périphérique Θ_{b1} à différentes localisations le long du câble électrique 10.

Cette unité de détermination 22 est configurée pour déterminer la température de conducteur Θ_{cond}, i.e. la température du conducteur électrique 12.

Cette détermination est réalisée de manière non-invasive et non-destructrice. Ainsi, aucun composant n'est inséré sous les couches de matériau ou à proximité du conducteur électrique 12 pour déterminer sa température de conducteur Θ_{cond}. De plus, aucune couche de matériau du câble électrique 10 n'est endommagée ou transpercée pour réaliser cette détermination. Aucun composant tiers n'est intégré à la fabrication du câble électrique 10 telle qu'une fibre optique ou un capteur dans l'une des couches du câble électrique 10 ou bien entre ces couches de matériau.

Ceci permet de réaliser une détermination de la température de conducteur sur un câble électrique existant, par exemple déjà installé in situ, sans avoir la nécessité de l'endommager ou d'y insérer un quelconque outil de mesure.

On considère ici que l'adjonction de composants de mesure ou de couches de matériau supplémentaires n'est pas invasif ou destructif.

L'unité de détermination 22 utilise un modèle physique permettant de déterminer la température de conducteur Θ_{cond} en fonction de la température périphérique Θ_{b1} mesurée par le ou les capteurs de température 20.

En référence à la figure 5, la diffusion de la chaleur au travers du câble électrique 10 est représentée sous forme de schéma pour illustrer le modèle physique utilisé par l'unité de détermination 22.

Ce modèle physique repose sur le fait que la diffusion de la chaleur au travers des couches d'un câble électrique suit un comportement proche de celui de la circulation d'un courant au sein d'un circuit électrique comportant une résistance électrique.

Ainsi, le modèle physique établit une relation entre la résistance thermique T1 de ladite au moins une couche de matériau. La résistance thermique T₁ peut correspondre à la résistance thermique de l'une ou plusieurs des couches de matériau. Dans l'exemple de la figure 4, la résistance thermique T₁ représente la résistance thermique de l'ensemble des première 14 et deuxième 16 couches de matériau. Pour ce modèle physique, les première 14 et deuxième 16 couches de matériau forment donc une seule et même couche de matériau présentant une résistance thermique de couche, appelée T₁.

Le passage du courant à l'intérieur du conducteur génère un échauffement induisant un flux thermique W_{c}.

Dans ce modèle physique, la différence de tension ΔU aux bornes d'une résistance électrique est rapprochée d'une différence de température ΔΘ entre les surfaces interne et externe de ladite au moins une couche de matériau (i.e. aux bornes de cette couche de matériau).

Dans l'application du câble électrique 10, les températures aux bornes de la résistance thermique T1 sont d'une part la température du conducteur Θ_{cond} et d'autre part la température périphérique Θ_{b1}. Ainsi, la différence de température ΔΘ s'exprime comme suit : ΔΘ = Θ_{cond} - Θ_{b1}.

Selon ce modèle physique, une relation mathématique est établie entre flux thermique W_{c}, la résistance thermique T1 et la différence de température ΔΘ aux bornes de cette résistance thermique. Cette relation est la suivante : $ΔΘ = {T}_{1} {* W}_{c}$ avec
ΔΘ étant la différence de température ΔΘ entre les surfaces interne et externe de couche de ladite au moins une couche de matériau,
T₁ étant la résistance thermique de ladite au moins une couche de matériau, W_{c} étant le flux thermique généré par l'échauffement du conducteur.

La température de conducteur Θ_{cond} peut donc s'exprimer comme suit : ${Θ}_{cond} = {Θ}_{b 1} + {W}_{c} {* T}_{1}$ avec
Θ_{cond} étant la température de conducteur,
Θ_{b1} étant la température périphérique.

La résistance thermique T₁ de ladite au moins une couche de matériau est déterminée comme suit : ${T}_{1} = \frac{{ρ}_{T}}{2 \times π} ln \left(1+2∗\frac{{t}_{1}}{{d}_{c}}\right)$ avec
ρ_{T} étant la conductivité thermique de ladite au moins une couche de matériau,
d_{c} étant le diamètre interne de ladite au moins une couche de matériau,
t₁ étant l'épaisseur de ladite au moins une couche de matériau.

Le modèle physique comprend deux modes de détermination de la température de conducteur Θ_{cond}.

Dans le premier mode de détermination, le système de détermination 100 comprend un dispositif de mesure de l'intensité électrique I_{cond} d'un courant électrique circulant dans ledit au moins un conducteur électrique 12.

Le dispositif de mesure est un dispositif non-invasif, notamment du type d'un enroulement de Rogowski.

Dans le deuxième mode de détermination, le système de détermination 100 comprend au moins une couche additionnelle 24 de matériau et au moins un capteur de température additionnel 26.

Ce deuxième mode de détermination permet de s'affranchir de l'utilisation de l'intensité électrique I_{cond} du courant circulant dans le conducteur.

Ladite au moins une couche additionnelle de matériau 24 est disposée autour de ladite au moins une couche de matériau. La ou les couches additionnelles de matériau 24 recouvre ledit au moins un capteur de température 22, tel que visible sur la figure 6.

Ces deux modes de détermination peuvent être utilisés pour la détermination de la température de conducteur Θ_{cond} selon différentes modélisations d'un câble électrique 10. Ces différentes modélisations peuvent impliquer différentes hypothèses (e.g. prise en compte ou non de pertes diélectriques) ou bien différentes configurations du câble électrique 10.

L'unité de détermination 22 est configuré pour mettre en œuvre le premier et/ou le deuxième modes de détermination. L'unité de détermination 22 est configurée pour déterminer la température de conducteur Θ_{cond} selon l'une ou plusieurs modélisations, notamment l'une ou plusieurs parmi les modélisations présentées ci-après.

### Câble électrique sans écran et sans prise en compte des pertes diélectriques

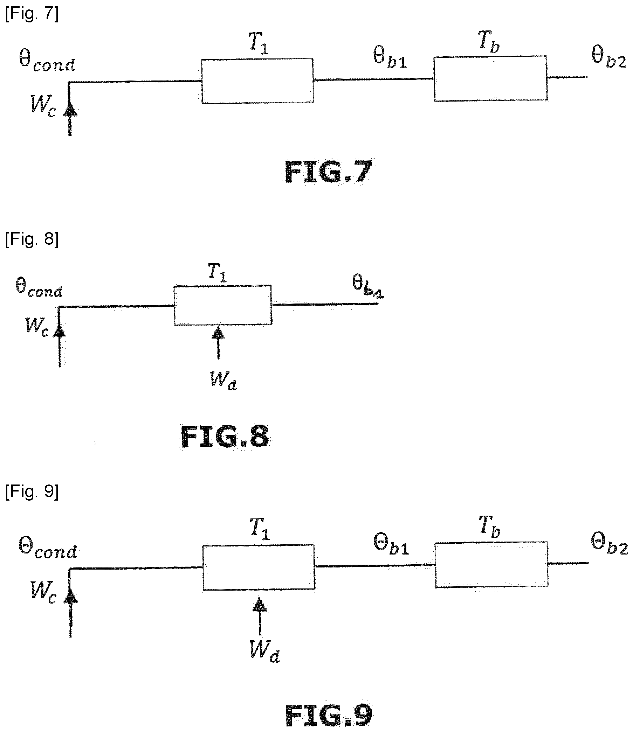

L'unité de détermination 22 est configurée pour déterminer la température de conducteur Θ_{cond} selon une première et une deuxième modélisations respectivement illustrées sur les figures 5 et 7.

Plus spécifiquement, l'unité de détermination 22 est configurée pour la température de conducteur Θ_{cond} selon la première modélisation au moyen du premier mode de détermination. L'unité de détermination 22 est configurée pour la température de conducteur Θ_{cond} selon la deuxième modélisation au moyen du deuxième mode de détermination.

Dans les première et deuxième modélisations, les pertes diélectriques dans ladite au moins une couche de matériau ne sont pas prises en compte ou considérées comme minimes.

Dans ces première et deuxième modélisations, le câble électrique 10 est dépourvu d'écran.

La première modélisation s'applique à câble électrique 10 comprenant un conducteur électrique 12 et une ou plusieurs couches de matériau entourant le conducteur électrique 12. Un ou plusieurs capteurs de température 20 sont disposés sur la surface externe 18 de ladite au moins une couche de matériau.

Le câble électrique 10 selon la figure 4 est un exemple compatible avec cette première modélisation.

Tel qu'indiqué ci-avant, la température de conducteur Θ_{cond} peut s'exprimer comme suit : ${Θ}_{cond} = {Θ}_{b 1} + {W}_{c} {* T}_{1}$ avec
Θ_{cond} étant la température de conducteur,
Θ_{b1} étant la température périphérique,
T₁ étant la résistance thermique de ladite au moins une couche de matériau,
W_{c} étant le flux thermique généré par l'échauffement du conducteur.

Selon le premier mode de détermination, on exprime le flux thermique Wc dû à l'échauffement du conducteur électrique 12 comme suit : ${W}_{c} = {R}_{c} {{* I}_{cond}}^{2}$
R_{c} étant la résistance électrique du conducteur électrique,
I_{cond} étant l'intensité du courant circulant le long du conducteur électrique.

La température de conducteur Θ_{cond} selon le premier mode de détermination, i.e. fonction de l'intensité du conducteur électrique, s'exprime donc comme suit : ${Θ}_{cond} = {Θ}_{b 1} + {R}_{c} {{* I}_{cond}}^{2} {* T}_{1}$

Tel que vu précédemment, la résistance électrique Rc du conducteur électrique s'exprime comme suit : ${R}_{c} = {R}_{0} \times \left(1+{α}_{20}\times \left({Θ}_{cond}-20\right)\right) \times \left(1+{y}_{s}+{y}_{p}\right)$ avec
R₀ étant la résistance en courant continu du conducteur à 20°C, en Ohm,
Yₛ étant le facteur d'effet de peau, sans unité,
Yₚ étant le facteur d'effet de proximité, sans unité,
α₂₀ étant le coefficient de résistivité électrique, en K⁻¹ (i.e. par kelvin).Les paramètres R₀, Yₛ, Yₚ et α₂₀ sont des valeurs liées à la structure et la nature du conducteur électrique.

La température de conducteur Θ_{cond} peut ainsi s'exprimer comme suit : ${Θ}_{cond} = \frac{{Θ}_{b 1} - {R}_{0}^{2} \times {T}_{1} \times \left(1-20\times {α}_{20}\right)}{1 - {R}_{0} \times {I}_{cond}^{2} \times {α}_{20} \times {T}_{1}}$

Selon le deuxième mode de détermination, i.e. sans l'intensité du conducteur I_{cond}, l'unité de détermination 100 comprend une structure additionnelle illustrée en figure 6. Ainsi, le système de détermination 100 comprend au moins une couche additionnelle 24 de matériau et au moins un capteur de température additionnel 26.

Ladite au moins une couche additionnelle de matériau 24 présente une résistance thermique additionnelle T_{b}.

Ladite au moins une couche additionnelle de matériau 24 est par exemple au moins une couche isolante électriquement.

Le matériau de ladite au moins une couche additionnelle de matériau 24 présente de préférence une résistance thermique entre 0,001 m².K/W et 0,1 m².K/W. Dans cette plage de résistance thermique, ladite au moins une couche de matériau 24permet d'éviter la surchauffe du conducteur tout permettant une différence de température suffisamment grande pour être mesurée.

Ledit au moins un capteur de température additionnel 26 permet de mesurer une température périphérique additionnelle Θ_{b2} au niveau de la surface externe 28 de ladite au moins une couche additionnelle de matériau 24.

Le système de détermination 100 peut comprendre une pluralité de capteurs de température additionnels 26 répartis autour de l'axe longitudinal A dans un même plan transversal à l'axe longitudinal A. Dans l'exemple de la figure 6, le système de détermination 100 comprend 9 capteurs de température additionnels 26.

Le ou les capteurs de température additionnels 26 sont connectés à l'unité de détermination 22 de manière à communiquer la température périphérique additionnelle Θ_{b2} à cette unité de détermination 22.

De préférence, les capteurs de température additionnels 26 sont équirépartis autour de l'axe longitudinal A. Le système de détermination 100 peut prévoir un ou plusieurs capteurs de température additionnels 26 répartis en outre le long de l'axe longitudinal A de manière à mesurer la température périphérique additionnelle Θ_{b2} à différentes localisations le long du câble électrique 10.

De préférence, le nombre et/ou la position angulaire et/ou la position longitudinale des capteurs de température 20 sont respectivement identiques au nombre et/ou à la position angulaire et/ou la position longitudinale des capteurs de température additionnels 26.

Le câble électrique 10 équipé de ladite au moins une couche additionnelle de matériau 24 et dudit au moins un capteur de température additionnel 26 est modélisé par une deuxième modélisation sur la figure 7. Ladite au moins une couche additionnelle de matériau 24 est considérée comme une résistance de valeur T_{b} en série avec la résistance de valeur T₁ correspondant à ladite au moins une couche de matériau.

Selon ce deuxième mode de détermination, on exprime le flux thermique W_{c} commet suit : ${W}_{c} = \frac{\left({Θ}_{b 1}-{Θ}_{b 2}\right)}{{T}_{b}}$

La température de conducteur peut ainsi être exprimée comme suit : ${θ}_{cond} = {θ}_{b 1} + \frac{\left({θ}_{b 1}-{θ}_{b 2}\right)}{{T}_{b}} \times {T}_{1}$

La détermination de la température de conducteur Θ_{cond} peut ainsi être déterminée sans nécessiter la valeur de l'intensité du courant circulant dans le conducteur électrique 12. Cette détermination est rendue possible par l'adjonction d'une couche additionnelle et d'un capteur additionnel.

### Câble électrique sans écran et avec prise en compte des pertes diélectriques

L'unité de détermination 22 est configurée pour déterminer la température de conducteur Θ_{cond} selon une troisième et une quatrième modélisations respectivement illustrées sur les figures 8 et 9.

Plus spécifiquement, l'unité de détermination 22 est configurée pour déterminer la température de conducteur Θ_{cond} selon la troisième modélisation au moyen du premier mode de détermination. L'unité de détermination 22 est configurée pour la température de conducteur Θ_{cond} selon la quatrième modélisation au moyen du deuxième mode de détermination.

Dans les troisième et quatrième modélisations, les pertes diélectriques dans ladite au moins une couche de matériau sont prises en compte.

Dans ces troisième et quatrième modélisations, le câble électrique 10 est dépourvu d'écran.

Dans ces troisième et quatrième modélisations, les pertes diélectriques dans ladite au moins une couche de matériau sont considérées comme un flux thermique de perte W_{d} au niveau de la résistance de valeur T₁ correspondant à ladite au moins une couche de matériau. Ce flux thermique de perte W_{d} est visible sur les figures 8 et 9.

La troisième modélisation s'applique à câble électrique 10 comprenant un conducteur électrique 12 et une ou plusieurs couches de matériau entourant le conducteur électrique 12. Un ou plusieurs capteurs de température 20 sont disposés sur la surface externe 18 de ladite au moins une couche de matériau.

Le câble électrique 10 selon la figure 4 est un exemple compatible avec cette troisième modélisation.

Selon le premier mode de détermination, la température de conducteur Θ_{cond} peut s'exprimer comme suit en fonction de l'intensité électrique I_{cond} : ${θ}_{cond} = {θ}_{b 1} + {T}_{1} \times \left({W}_{c}-\frac{{W}_{d}}{2}\right)$

Cette température de conducteur Θ_{cond} peut également s'exprimer comme suit en décomposant R_{c} tel que décrit ci-avant : ${θ}_{cond} = \frac{{θ}_{b 1} - \left({R}_{0}{I}_{cond}^{2}\right) {T}_{1} \left(1-20\times {α}_{20}\right) + \frac{1}{2} {T}_{1} {W}_{d}}{1 - {R}_{0} {I}_{cond}^{2} {α}_{20} {T}_{1}}$

Selon le deuxième mode de détermination, i.e. sans l'intensité du conducteur I_{cond}, l'unité de détermination 100 comprend une structure additionnelle telle qu'illustrée en figure 6. Ainsi, le système de détermination 100 comprend au moins une couche additionnelle 24 de matériau et au moins un capteur de température additionnel 26.

Le câble électrique 10 équipé de ladite au moins une couche additionnelle de matériau 24 et dudit au moins un capteur de température additionnel 26 est modélisé par une quatrième modélisation sur la figure 9.

Ladite au moins une couche additionnelle de matériau 24 est considérée comme une résistance de valeur T_{b} en série avec la résistance de valeur T₁ correspondant à ladite au moins une couche de matériau.

Selon ce deuxième mode de détermination, on exprime la température de conducteur Θ_{cond} comme suit : ${θ}_{cond} = {θ}_{b 1} + {T}_{1} \times \left(\frac{\left({θ}_{b 1}-{θ}_{b 2}\right)}{{T}_{b}}-\frac{{W}_{d}}{2}\right)$

L'équation précédente est obtenue en considérant les équations suivantes : ${θ}_{cond} = {θ}_{b 1} + {T}_{1} \times \left({W}_{c}+\frac{{W}_{d}}{2}\right)$ et ${θ}_{b 1} = {θ}_{b 2} + {T}_{b} \times \left({W}_{d}+{W}_{c}\right)$

Le flux thermique de perte W_{d} est déterminé en fonction de la tension appliquée au conducteur électrique 12, de la fréquence de la tension appliquée au conducteur électrique 12 et des caractéristiques diélectriques de ladite au moins une couche de matériau.

### Câble électrique avec écran et prise en compte des pertes diélectriques

L'unité de détermination 22 est en outre configurée pour déterminer la température de conducteur Θ_{cond} dans une configuration du câble électrique 10 comprenant un écran 17.

Tel qu'illustré en figure 10, le câble électrique 10 comprend un conducteur électrique 12, une ou plusieurs couches de matériau entourant le conducteur électrique 12 et un écran 17 entourant lesdites couches de matériau.

Lesdites couches de matériau sont par exemple une couche diélectrique 30 entourant le conducteur électrique 12 et une couche isolante 32 disposée entre la couche diélectrique 30 et l'écran 17.

Le câble électrique 10 comprend également une couche externe 34, par exemple une gaine externe, définissant une surface externe 38 de la couche externe 34. La couche externe 34 peut comprendre une pluralité de couches de matériau.

La couche externe 34 présente une résistance thermique T₃.

Un ou plusieurs capteurs de température 20 sont disposés sur la surface externe 38 de la couche externe 34.

Des pertes dans l'écran 17 sont modélisées par un flux thermique d'écran Wₛ. Ces pertes sont dues à un échauffement par effet joule dans l'écran 17.

La détermination du flux thermique d'écran Wₛ requiert une mesure invasive sur le câble électrique 10. Pour s'affranchir d'exprimer la température de conducteur Θ_{cond} en fonction du flux thermique Wₛ, il est ici proposé de combiner les premier et deuxième modes de détermination vu précdemment. En d'autres termes, il est prévu ici d'exprimer la température de conducteur Θ_{cond} en fonction de l'intensité I_{cond} de la tension circulant dans le conducteur électrique 12 et d'utiliser une structure additionnelle comprenant au moins une couche additionnelle de matériau 24 et au moins un capteur de température additionnel 26, tel que visible sur la figure 10.

Ladite au moins une couche additionnelle de matériau 24 présente une résistance thermique T_{b}.

Une cinquième modélisation est illustrée à la figure 11 prenant en compte les pertes d'écran (flux thermique Wₛ) ainsi que les pertes diélectriques dans ladite au moins une couche (flux thermique W_{d}) et comprenant trois résistances en série pour modéliser les résistances thermiques de ladite au moins une couche de matériau (T₁), de la gaine externe 34 (T₃) et de ladite au moins une couche additionnelle de matériau 24 (T_{b}).

Dans cette cinquième modélisation, la température de conducteur Θ_{cond} s'exprime comme suit : ${θ}_{cond} = \frac{{θ}_{b 1} + {T}_{3} \frac{{Δθ}_{b}}{{T}_{b}} + \frac{1}{2} {T}_{1} {W}_{d} - \left({R}_{0}{I}^{2}\right) {T}_{1} \left(1-20\times {α}_{20}\right)}{1 - {R}_{0} {I}_{cond}^{2} {α}_{20} {T}_{1}}$
Θ_{b1} étant la température périphérique mesurée par ledit au moins un capteur de température 20,
Θ_{b2} étant la température périphérique additionnelle mesurée par ledit au moins un capteur de température additionnel 26,
ΔΘ étant la différence de température Θ_{b1} - O_{b2},
T₁ étant la résistance thermique de ladite au moins une couche de matériau,
T_{b} étant la résistance thermique de ladite au moins une couche additionnelle de matériau 24,
T₃ étant la résistance thermique de couche externe 34,
R₀ étant la résistance en courant continu du conducteur à 20°C, en Ohm,
Yₛ étant le facteur d'effet de peau, sans unité,
Yₚ étant le facteur d'effet de proximité, sans unité,
α₂₀ étant le coefficient de résistivité électrique, en K⁻¹ (i.e. par kelvin).

L'unité de détermination 22 est ainsi capable de déterminer la température de conducteur Θ_{cond} indépendamment du flux thermique d'écran Wₛ.

Cette expression de la température de conducteur Θ_{cond} est obtenue en considérant que : ${θ}_{cond} = {θ}_{surf} + n \left({W}_{c}+{W}_{s}+{W}_{d}\right) {T}_{3} + \left({W}_{c}+\frac{{W}_{d}}{2}\right) {T}_{1}$ $n \left({W}_{c}+{W}_{s}+{W}_{d}\right) = \frac{{Δθ}_{b}}{{T}_{b}}$ ${θ}_{conducteur} = {θ}_{b 1} + {T}_{3} \frac{{Δθ}_{b}}{{T}_{b}} + \left({W}_{c}+\frac{{W}_{d}}{2}\right) {T}_{1}$
avec Θ_{surf} étant la température périphérique au niveau de la surface externe du câble électrique 10, et
n étant le nombre de conducteurs électriques 12.

Tel que détaillé précédemment, la résistance thermique T₁ est déterminée comme suit : ${T}_{1} = \frac{{ρ}_{T}}{2 \times π} ln \left(1+2∗\frac{{t}_{1}}{{d}_{c}}\right)$

La résistance thermique T₃ de la couche externe 34 est déterminée comme suit : ${T}_{3} = \frac{{ρ}_{T}}{2 \times π} ln \left(1+2∗\frac{{t}_{3}}{{D}_{a}}\right)$ avec
t₃ étant l'épaisseur de la couche externe 34,
Dₐ étant le diamètre interne de la couche externe 34.

Selon une sixième modélisation illustrée en figure 12, l'unité de détermination est également configurée pour déterminer la température de conducteur Θ_{cond} de manière indépendante du milieu environnant, notamment de la température de ce milieu environnant.

Cette sixième modélisation s'applique à la même configuration de câble électrique 10 que la cinquième modélisation. En d'autres termes, la sixième modélisation s'applique à un câble électrique du type de celui de la figure 10 avec une structure additionnelle et un écran 17.

Selon le milieu environnant, la chaleur va être plus ou moins bien évacuée du câble électrique 10. Le milieu environnant est modélisé par une couche de matériau avec une certain résistance thermique T₅ et une température θₐ correspondant à la température ambiante du milieu environnant.

La température de conducteur Θ_{cond} peut s'exprimer ainsi : ${θ}_{cond} = {θ}_{a} + \left({W}_{c}+\frac{{W}_{d}}{2}\right) {T}_{1} + n \left({W}_{c}+{W}_{d}+{W}_{s}\right) {T}_{3} + n \left({W}_{c}+{W}_{d}+{W}_{s}\right) {T}_{b} + n \left({W}_{c}+{W}_{d}+{W}_{s}\right) {T}_{5}$

La différence de température Θ_{b1} - Θ_{b2} de part et d'autre de la couche additionnelle de matériau 24 permet d'exprimer la température de conducteur Θ_{cond} comme suit : ${θ}_{cond} = {θ}_{b 1} + \left({W}_{c}+\frac{{W}_{d}}{2}\right) {T}_{1} + n \left({W}_{c}+{W}_{d}+{W}_{s}\right) {T}_{b}$ et ${θ}_{b 1} - {θ}_{b 2} = n \left({W}_{c}+{W}_{d}+{W}_{s}\right) {T}_{b}$

La température de conducteur Θ_{cond} peut ainsi être déterminée par l'unité de détermination 22 indépendamment du milieu environnant.

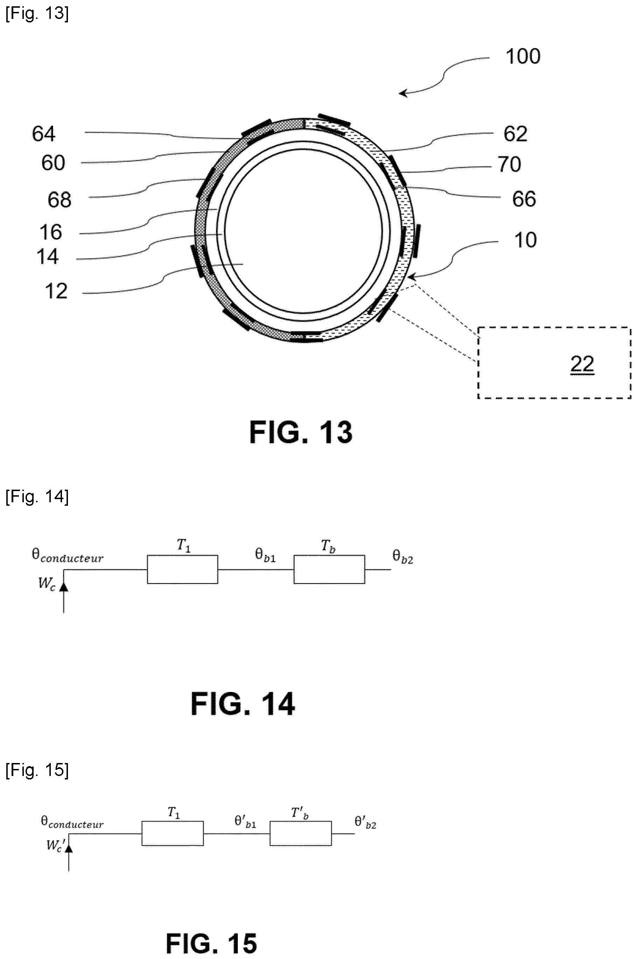

En lien avec les figures 13 à 19, l'unité de détermination 22 est en outre configurée pour déterminer la température de conducteur Θ_{cond} selon une septième et une huitième modélisations.

Dans septième et huitième modélisations, l'unité de détermination 22 est configurée pour déterminer la température de conducteur Θ_{cond} sans nécessiter de détermination du flux thermique W_{c} généré par la circulation d'un courant électrique dans le conducteur électrique.

Pour ces septième et huitième modélisations, le système de détermination 100 est semblable à celui de la figure 6 avec pour différence que ladite au moins une couche additionnelle 24 comprend une première 60 et une deuxième 62 portions de couche additionnelle, tel qu'illustré en figure 13.

La première portion de couche additionnelle 60 présente une première résistance thermique additionnelle T_{b} La deuxième portion de couche additionnelle 62 présente une deuxième résistance thermique additionnelle T'_{b}. Les première T_{b} et deuxième T'_{b} résistances thermiques additionnelles sont différentes.

Cette différence entre les première T_{b} et deuxième T'_{b} résistances thermiques additionnelles peut être obtenue par utilisation d'un matériau différent et/ou d'une ou plusieurs caractéristiques géométriques différentes entre les première 60 et deuxième 62 portions de couche additionnelle. Un exemple de caractéristique géométrique différente est une épaisseur différente, prise le long d'un axe perpendiculaire à l'axe longitudinal d'extension A du conducteur électrique.

Cette différence entre les première T_{b} et deuxième T'_{b} résistances thermiques additionnelles permet de construire deux équations différentes ayant pour inconnue la température de conducteur Θ_{cond}. Il est ainsi possible de s'affranchir de la connaissance du flux thermique W_{c}

Une pluralité de premiers capteurs de température 64 sont disposés sur la surface externe 18 de ladite au moins une couche de matériau, entre ladite surface externe 18 et la première portion de couche additionnelle 60. La pluralité de premiers capteurs de température 64 permettent de mesurer une première température périphérique Θ_{b1} au niveau de la surface externe 18 de ladite au moins une couche de matériau.

Une pluralité de deuxièmes capteurs de température 66 sont disposés sur la surface externe 18 de ladite au moins une couche de matériau, entre ladite surface externe 18 et la deuxième portion de couche additionnelle 62. La pluralité de deuxièmes capteurs de température 66 permettent de mesurer une deuxième température périphérique Θ'_{b1} au niveau de la surface externe 18 de ladite au moins une couche de matériau.

Une pluralité de premiers capteurs de température additionnels 68 sont disposés sur une surface externe de la première portion de couche additionnelle 60 pour mesurer une première température périphérique additionnelle Θ_{b2}.

Une pluralité de deuxièmes capteurs de température additionnels sont disposés sur une surface externe de la deuxième portion de couche additionnelle pour mesurer une deuxième température périphérique additionnelle Θ'_{b2}.

L'unité de détermination 22 est configurée pour déterminer la température de conducteur Θ_{cond} en outre en fonction de la première T_{b} et de la deuxième T'_{b} résistances thermiques additionnelles ainsi que de la première Θ_{b2} et de la deuxième Θ'_{b2} températures périphériques additionnelles.

### Câble électrique sans écran sans prise en compte des pertes diélectriques

La septième modélisation est illustrée dans les figures 14 à 16.

Dans la septième modélisation, la température de conducteur Θ_{cond} s'exprime comme ci-après: ${θ}_{conducteur} = \frac{\left({θ}_{b 1}{T}_{b}-θ{′}_{b 1}T{′}_{b}\right) \times \left(\left({θ}_{b 1}-{θ}_{b 2}\right)\right)}{{T}_{b} \left(θ{′}_{b 1}-θ{′}_{b 2}\right) - {T}_{b}^{′} \left({θ}_{b 1}-{θ}_{b 2}\right)}$

Cette équation est obtenue via les développements suivant : $\frac{\left({θ}_{conducteur}-{θ}_{b 1}\right)}{{T}_{1}} = \frac{\left({θ}_{b 1}-{θ}_{b 2}\right)}{{T}_{b}}$ $\frac{\left({θ}_{conducteur}-θ{′}_{b 1}\right)}{{T}_{1}} = \frac{\left(θ{′}_{b 1}-θ{′}_{b 2}\right)}{T {′}_{b}}$ $\left({θ}_{conducteur}-{θ}_{b 1}\right) \frac{{T}_{b}}{\left({θ}_{b 1}-{θ}_{b 2}\right)} = \left({θ}_{conducteur}-θ{′}_{b 1}\right) \frac{T {′}_{b}}{\left(θ{′}_{b 1}-θ{′}_{b 2}\right)}$ ${θ}_{conducteur} = \frac{{θ}_{b 1} {T}_{b} - θ {′}_{b 1} T {′}_{b}}{\left(θ{′}_{b 1}-θ{′}_{b 2}\right)} / \left(\frac{{T}_{b}}{\left({θ}_{b 1}-{θ}_{b 2}\right)}-\frac{T {′}_{b}}{\left(θ{′}_{b 1}-θ{′}_{b 2}\right)}\right)$

### Câble électrique sans écran avec prise en compte des pertes diélectriques

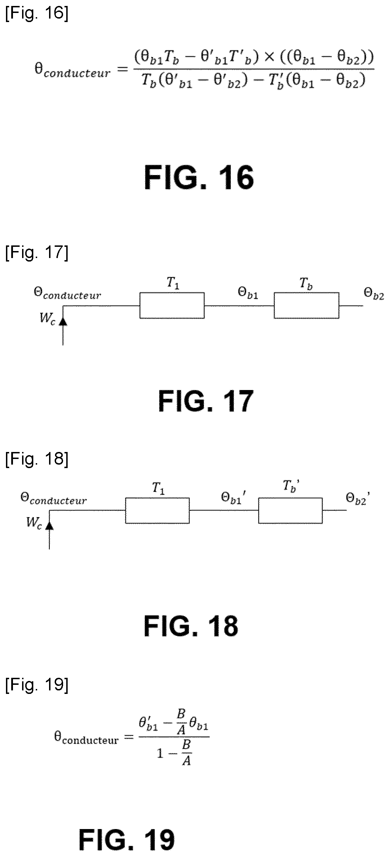

La huitième modélisation est illustrée dans les figures 17 à 19.

Dans la huitième modélisation, la température de conducteur Θ_{cond} s'exprime comme ci-après: ${θ}_{conducteur} = \frac{{θ}_{b 1}^{′} - \frac{B}{A} {θ}_{b 1}}{1 - \frac{B}{A}}$

Cette équation est obtenue via les développements suivant : ${θ}_{conducteur} = {θ}_{b 1} + {T}_{1} \times \left(\frac{\left({θ}_{b 1}-{θ}_{b 2}\right)}{{T}_{b}}-\frac{{W}_{d}}{2}\right)$ ${θ}_{conducteur} = {θ}_{b 1} ′ + {T}_{1} \times \left(\frac{\left({θ}_{b 1}′-{θ}_{b 2}′\right)}{{T}_{b} ′}-\frac{{W}_{d}}{2}\right)$ $A = \left(\frac{\left({θ}_{b 1}-{θ}_{b 2}\right)}{{T}_{b}}-\frac{{W}_{d}}{2}\right)$ $B = \left(\frac{\left({θ}_{b 1}′-{θ}_{b 2}′\right)}{{T}_{b} ′}-\frac{{W}_{d}}{2}\right)$
Θ_{b1}' étant la première température périphérique au niveau de la surface externe 18 de ladite au moins une couche de matériau,
Θ_{b2}' étant la deuxième température périphérique additionnelle,
T_{b}' étant la deuxième résistance thermique additionnelle.

## Revendications

1. Système de détermination (100) d'une quantité d'émissions de dioxyde de carbone résultant de l'échauffement d'un conducteur électrique d'un câble électrique par effet Joule, ledit système de détermination comprenant :
- un câble électrique (10) comprenant au moins un conducteur électrique (12) et au moins une couche de matériau (14, 16, 17, 30, 32, 34) entourant ledit au moins un conducteur,
- une unité de mesure (110) associée au câble électrique, ladite unité de mesure comprenant au moins un capteur de température (20) et un dispositif de mesure de l'intensité électrique I_{cond} (112) d'un courant électrique circulant dans le conducteur électrique,
- une unité de calcul (120) configurée pour communiquer des informations avec l'unité de mesure, l'unité de calcul étant configurée pour déterminer la température de conducteur Θ_{cond} au moyen dudit au moins un capteur de température, ladite unité de calcul étant en outre configurée pour déterminer une quantité d'émissions de dioxyde de carbone résultant de l'échauffement du conducteur électrique par effet joule en fonction de la température de conducteur Θ_{cond} et de l'intensité électrique I_{cond} dans le conducteur électrique.

2. Système de détermination (100) selon la revendication 1 dans lequel l'unité de calcul est configurée pour déterminer une résistance électrique R_{c} du conducteur électrique en fonction de la température de conducteur Θ_{cond}.

3. Système de détermination (100) selon la revendication 2, dans lequel l'unité de calcul est configurée pour déterminer une perte de puissance PₒL en fonction de l'intensité électrique I_{cond} dans le conducteur électrique et de la résistance électrique R_{c} du conducteur électrique, l'unité de calcul étant configurée pour déterminer la quantité d'émissions de dioxyde de carbone en fonction de ladite perte de puissance PₒL.

4. Système de détermination (100) selon l'une quelconque des revendications précédentes, comprenant en outre un boîtier de mesure monté sur le câble électrique, ledit boîtier de mesure comprenant au moins l'un parmi ledit au moins un capteur de température et le dispositif de mesure de l'intensité électrique I_{cond}.

5. Système de détermination (100) selon la revendication 4, dans lequel le boîtier de mesure comprend en outre l'unité de calcul.

6. Système de détermination (100) selon la revendication 4 ou 5, dans lequel le boîtier de mesure configuré pour être monté de manière amovible sur le câble électrique.

7. Système de détermination (100) selon la revendication 6, dans lequel le boîtier de mesure comprend un dispositif de fixation au câble électrique.

8. Système de détermination (100) selon l'une quelconque des revendications précédentes, dans lequel ledit au moins un capteur de température est disposé sur une surface externe de ladite au moins une couche de matériau pour mesurer une température périphérique Θ_{b1} au niveau de la surface externe de ladite au moins une couche de matériau, l'unité de calcul étant configurée pour déterminer la température de conducteur Θ_{cond} en fonction de la température périphérique Θ_{b1}.

9. Système de détermination (100) selon la revendication 8, comprenant en outre :
- une couche additionnelle de matériau disposée autour de ladite au moins une couche de matériau et recouvrant ledit au moins un capteur de température,
- au moins un capteur de température additionnel disposé sur une surface externe de ladite couche de matériau additionnelle pour mesurer une température périphérique additionnelle Θ_{b2} au niveau de la surface externe de ladite au moins une couche additionnelle de matériau.

10. Système de détermination (100) selon la revendication précédente, dans lequel l'unité de calcul est configurée pour déterminer la température de conducteur Θ_{cond} en fonction de la température périphérique Θ_{b1} et de la température périphérique additionnelle Θ_{b2}.

11. Système de détermination (100) selon la revendication 9 ou 10, dans lequel ladite au moins une couche additionnelle de matériau comprend :
- une première portion de couche additionnelle présentant une première résistance thermique additionnelle T_{b}, et
- une deuxième portion de couche additionnelle présentant une deuxième résistance thermique additionnelle T'_{b}, les première T_{b} et deuxième T'_{b} résistances thermiques additionnelles étant différentes,
et dans lequel ledit au moins un capteur de température additionnel comprend :
- au moins un premier capteur de température additionnel disposé sur une surface externe de la première portion de couche additionnelle pour mesurer une première température périphérique additionnelle O_{b2},
- au moins un deuxième capteur de température additionnel disposé sur une surface externe de la deuxième portion de couche additionnelle pour mesurer une deuxième température périphérique additionnelle Θ'_{b2},
l'unité de détermination étant configurée pour déterminer la température de conducteur Θ_{cond} en outre en fonction de la première T_{b} et de la deuxième T'_{b} résistances thermiques additionnelles ainsi que de la première Θ_{b2} et de la deuxième Θ'_{b2} températures périphériques additionnelles.

12. Système de détermination (100) selon la revendication précédente, dans lequel lesdites au moins une première et au moins une deuxième couches additionnelles sont disposées sur des secteurs angulaires différents autour du conducteur électrique.

13. Système de détermination (100) selon la revendication précédente, dans lequel ledit au moins un capteur de température (20) comprend :
- au moins un premier capteur disposé sur la surface externe (18) de ladite au moins une couche de matériau, entre ladite surface externe (18) et la première portion de couche additionnelle, pour mesurer une température périphérique Θ_{b1} au niveau de la surface externe de ladite au moins une couche de matériau,
- au moins un deuxième capteur disposé sur la surface externe (18) de ladite au moins une couche de matériau, entre ladite surface externe (18) et la deuxième portion de couche additionnelle, pour mesurer une température périphérique Θ'_{b1} au niveau de la surface externe de ladite au moins une couche de matériau.

14. Système de détermination (100) selon la revendication précédente, dans lequel l'unité de détermination est configurée pour déterminer la température de conducteur Θ_{cond} sur la base de l'équation suivante : ${θ}_{conducteur} = \frac{\left({θ}_{b 1}{T}_{b}-θ{′}_{b 1}T{′}_{b}\right) \times \left(\left({θ}_{b 1}-{θ}_{b 2}\right)\right)}{{T}_{b} \left(θ{′}_{b 1}-θ{′}_{b 2}\right) - {T}_{b}^{′} \left({θ}_{b 1}-{θ}_{b 2}\right)}$
T₁ étant la résistance thermique de ladite au moins une couche de matériau,
T_{b} étant la première résistance thermique additionnelle de la première couche additionnelle de matériau,
T'_{b} étant la deuxième résistance thermique additionnelle de la deuxième couche additionnelle de matériau,
Θ_{b1} est la température périphérique mesurée par ledit au moins un premier capteur de température,
Θ_{b2} est la température périphérique additionnelle mesurée par ledit au moins un premier capteur de température additionnel,
Θ'_{b1} est la température périphérique mesurée par ledit au moins un deuxième capteur de température,
Θ'_{b2} est la température périphérique additionnelle mesurée par ledit au moins un deuxième capteur de température additionnel.

15. Système de détermination (100) selon l'une quelconque des revendications 11 à 14, dans lequel les première et deuxième portions de couche additionnelle sont réalisées dans un matériau différent.

16. Système de détermination (100) selon l'une quelconque des revendications 11 à 15, dans lequel les première et deuxième portions de couche additionnelle présentent une épaisseur différente, prise perpendiculairement à un axe longitudinal d'extension du câble électrique (10).

## Patentansprüche

1. System zur Bestimmung (100) einer Menge von Kohlendioxidemissionen aus der Erwärmung eines elektrischen Leiters eines elektrischen Kabels durch Joule-Effekt, wobei das Bestimmungssystem umfasst:
- ein elektrisches Kabel (10) mit mindestens einem elektrischen Leiter (12) und mindestens einer Materialschicht (14, 16, 17, 30, 32, 34), die den mindestens einen Leiter umgibt,
- eine dem elektrischen Kabel zugeordnete Messeinheit (110), wobei die Messeinheit mindestens einen Temperaturaufnehmer (20) und eine Vorrichtung zur Messung der elektrischen Stromstärke I_{cond} (112) eines in dem elektrischen Leiter fließenden elektrischen Stroms umfasst,
- eine Recheneinheit (120), die so konfiguriert ist, dass sie Informationen mit der Messeinheit austauscht, wobei die Recheneinheit so konfiguriert ist, dass sie die Leitertemperatur Θ_{cond} mittels des mindestens einen Temperaturaufnehmers bestimmt, wobei die Recheneinheit ferner so konfiguriert ist, dass sie eine Menge von Kohlendioxidemissionen bestimmt, die aus der Erwärmung des elektrischen Leiters durch Joule-Effekt entstehen, und zwar in Abhängigkeit von der Leitertemperatur Θ_{cond} und der elektrischen Stromstärke I_{cond} im elektrischen Leiter.

2. Bestimmungssystem (100) nach Anspruch 1, wobei die Recheneinheit so konfiguriert ist, dass sie einen elektrischen Widerstand R_{c} des elektrischen Leiters in Abhängigkeit von der Leitertemperatur Θ_{cond} bestimmt.

3. Bestimmungssystem (100) nach Anspruch 2, wobei die Recheneinheit so konfiguriert ist, dass sie einen Leistungsverlust PₒL in Abhängigkeit von der elektrischen Stromstärke I_{cond} im elektrischen Leiter und dem elektrischen Widerstand R_{c} des elektrischen Leiters bestimmt, wobei die Recheneinheit so konfiguriert ist, dass sie die Menge der Kohlendioxidemissionen in Abhängigkeit von dem genannten Leistungsverlust PₒL bestimmt.

4. Bestimmungssystem (100) nach einem der vorstehenden Ansprüche, das ferner ein am elektrischen Kabel angebrachtes Messgerät umfasst, wobei das Messgerät mindestens einen der mindestens einen Temperaturaufnehmer und die Vorrichtung zur Messung der elektrischen Stromstärke I_{cond} umfasst.

5. Bestimmungssystem (100) nach Anspruch 4, wobei das Messgerät ferner die Recheneinheit umfasst.

6. Bestimmungssystem (100) nach Anspruch 4 oder 5, wobei das Messgerät so konfiguriert ist, dass es abnehmbar am elektrischen Kabel angebracht werden kann.

7. Bestimmungssystem (100) nach Anspruch 6, wobei das Messgerät eine Vorrichtung zur Befestigung am elektrischen Kabel umfasst.

8. Bestimmungssystem (100) nach einem der vorstehenden Ansprüche, wobei der mindestens eine Temperaturaufnehmer an einer Außenfläche der mindestens einen Materialschicht angeordnet ist, um eine Oberflächentemperatur Θ_{b1} an der Außenfläche der mindestens einen Materialschicht zu messen, wobei die Recheneinheit so konfiguriert ist, dass sie die Leitertemperatur Θ_{cond} in Abhängigkeit von der Oberflächentemperatur Θ_{b1} bestimmt.

9. Bestimmungssystem (100) nach Anspruch 8, das ferner umfasst:
- eine zusätzliche Materialschicht, die um die mindestens eine Materialschicht herum angeordnet ist und den mindestens einen Temperaturaufnehmer überdeckt,
- mindestens einen zusätzlichen Temperaturaufnehmer, der an einer Außenfläche der zusätzlichen Materialschicht angeordnet ist, um eine zusätzliche Oberflächentemperatur Θ_{b2} an der Außenfläche der genannten mindestens einen zusätzlichen Materialschicht zu messen.

10. Bestimmungssystem (100) nach dem vorstehenden Anspruch, wobei die Recheneinheit so konfiguriert ist, dass sie die Leitertemperatur Θ_{cond} in Abhängigkeit von der Oberflächentemperatur Θ_{b1} und der zusätzlichen Oberflächentemperatur Θ_{b2} bestimmt.

11. Bestimmungssystem (100) nach Anspruch 9 oder 10, wobei die mindestens eine zusätzliche Materialschicht umfasst:
- einen ersten Abschnitt einer zusätzlichen Schicht mit einem ersten zusätzlichen Wärmewiderstand T_{b} und
- einen zweiten Abschnitt einer zusätzlichen Schicht mit einem zweiten zusätzlichen Wärmewiderstand T'_{b}, wobei der erste zusätzliche Wärmewiderstand T_{b} und der zweite zusätzliche Wärmewiderstand T'_{b} unterschiedlich sind,
und wobei der mindestens eine zusätzliche Temperaturaufnehmer umfasst:
- mindestens einen ersten zusätzlichen Temperaturaufnehmer, der an einer Außenfläche des ersten Abschnitts der zusätzlichen Schicht angeordnet ist, um eine erste zusätzliche Oberflächentemperatur Θ_{b2} zu messen,
- mindestens einen zweiten zusätzlichen Temperaturaufnehmer, der an einer Außenfläche des zweiten Abschnitts der zusätzlichen Schicht angeordnet ist, um eine zweite zusätzliche Oberflächentemperatur Θ'_{b2} zu messen,
wobei die Bestimmungseinheit so konfiguriert ist, dass sie die Leitertemperatur Θ_{cond} außerdem in Abhängigkeit von dem ersten zusätzlichen Wärmewiderstand T_{b} und dem zweiten zusätzlichen Wärmewiderstand T'_{b} sowie von der ersten zusätzlichen Oberflächentemperatur Θ_{b2} und der zweiten zusätzlichen Oberflächentemperatur Θ'_{b2} bestimmt.

12. Bestimmungssystem (100) nach dem vorstehenden Anspruch, wobei die mindestens eine erste und die mindestens eine zweite zusätzliche Schicht in unterschiedlichen Winkelbereichen um den elektrischen Leiter herum angeordnet sind.

13. Bestimmungssystem (100) nach dem vorstehenden Anspruch, wobei der mindestens eine Temperaturaufnehmer (20) umfasst:
- mindestens einen ersten Aufnehmer, der an der Außenfläche (18) der mindestens einen Materialschicht zwischen der Außenfläche (18) und dem ersten Abschnitt der zusätzlichen Schicht angeordnet ist, um eine Oberflächentemperatur Θ_{b1} an der Außenfläche der mindestens einen Materialschicht zu messen,
- mindestens einen zweiten Aufnehmer, der an der Außenfläche (18) der mindestens einen Materialschicht zwischen der Außenfläche (18) und dem zweiten Abschnitt der zusätzlichen Schicht angeordnet ist, um eine Oberflächentemperatur Θ'_{b1} an der Außenfläche der mindestens einen Materialschicht zu messen.

14. Bestimmungssystem (100) nach dem vorstehenden Anspruch, wobei die Bestimmungseinheit so konfiguriert ist, dass sie die Leitertemperatur Θ_{cond} auf Grundlage der folgenden Gleichung bestimmt: ${θ}_{conducteur} = \frac{\left({θ}_{b 1}{T}_{b}-θ{′}_{b 1}T{′}_{b}\right) \times \left(\left({θ}_{b 1}-{θ}_{b 2}\right)\right)}{{T}_{b} \left(θ{′}_{b 1}-θ{′}_{b 2}\right) - T {′}_{b} \left({θ}_{b 1}-{θ}_{b 2}\right)}$
wobei T₁ der Wärmewiderstand der mindestens einen Materialschicht ist,
wobei T_{b} der erste zusätzliche Wärmewiderstand der ersten zusätzlichen Materialschicht ist,
wobei T'_{b} der zweite zusätzliche Wärmewiderstand der zweiten zusätzlichen Materialschicht ist,
Θ_{b1} ist die von dem mindestens einen ersten Temperaturaufnehmer gemessene Oberflächentemperatur,
Θ_{b2} ist die von dem mindestens einen ersten zusätzlichen Temperaturaufnehmer gemessene zusätzliche Oberflächentemperatur,
Θ'_{b1} ist die von dem mindestens einen zweiten Temperaturaufnehmer gemessene Oberflächentemperatur,
Θ'_{b2} ist die von dem mindestens einen zweiten zusätzlichen Temperaturaufnehmer gemessene zusätzliche Oberflächentemperatur.

15. Bestimmungssystem (100) nach einem der Ansprüche 11 bis 14, wobei der erste und der zweite Abschnitt der zusätzlichen Schicht aus unterschiedlichem Material bestehen.

16. Bestimmungssystem (100) nach einem der Ansprüche 11 bis 15, wobei der erste und der zweite Abschnitt der zusätzlichen Schicht eine unterschiedliche Dicke aufweisen, gemessen senkrecht zu einer verlängerten Längsachse des elektrischen Kabels (10).

## Claims

1. A determination system (100) for determination of a quantity of emissions of carbon dioxide resulting from the heating of an electrical conductor of an electrical cable by the Joule effect, said determination system comprising:
- an electrical cable (10) comprising at least one electrical conductor (12) and at least one layer of material (14, 16, 17, 30, 32, 34) surrounding said at least one conductor,
- a measurement unit (110) associated with the electrical cable, said measurement unit comprising at least one temperature sensor (20) and a device for measurement of the electrical intensity I_{cond} (112) of an electrical current circulating in the electrical conductor,
- a calculation unit (120) configured to communicate information with the measurement unit, the calculation unit being configured to determine the conductor temperature Θ_{cond} by means of said at least one temperature sensor, said calculation unit being further configured to determine a quantity of emissions of carbon dioxide resulting from the heating of the electrical conductor by the Joule effect as a function of the conductor temperature Θ_{cond} and the electrical intensity I_{cond} in the electrical conductor.

2. The determination system (100) according to claim 1, in which the calculation unit is configured to determine an electrical resistance R_{c} of the electrical conductor as a function of the conductor temperature Θ_{cond}.

3. The determination system (100) according to claim 2, in which the calculation unit is configured to determine a power loss PₒL as a function of the electrical intensity I_{cond} in the electrical conductor and the electrical resistance R_{c} of the electrical conductor, the calculation unit being configured to determine the quantity of emissions of carbon dioxide as a function of said power loss PₒL.

4. The determination system (100) according to any one of the preceding claims, further comprising a measurement module mounted on the electrical cable, said measurement module comprising at least one of the following: said at least one temperature sensor and the device for measurement of the electrical intensity I_{cond}.

5. The determination system (100) according to claim 4, in which the measurement module further comprising the calculation unit.

6. The determination system (100) according to claim 4 or 5, in which the measurement module is configured to be removably mounted on the electrical cable.

7. The determination system (100) according to claim 6, in which the measurement module comprises a device for fixing it to the electrical cable.

8. The determination system (100) according to any one of the preceding claims, in which said at least one temperature sensor is disposed on an external surface of said at least one layer of material to measure a peripheral temperature Θ_{b1} at the level of the external surface of said at least one layer of material, the calculation unit being configured to determine the conductor temperature Θ_{cond} as a function of the peripheral temperature Θ_{b1}.

9. The determination system (100) according to claim 8, further comprising:
- an additional layer of material disposed around said at least one layer of material and covering said at least one temperature sensor,
- at least one additional temperature sensor disposed on an external surface of said additional layer of material to measure an additional peripheral temperature Θ_{b2} at the level of the external surface of said at least one additional layer of material.

10. The determination system (100) according to the preceding claim, in which the calculation unit is configured to determine the conductor temperature Θ_{cond} as a function of the peripheral temperature Θ_{b1} and the additional peripheral temperature Θ_{b2}

11. The determination system (100) according to claim 9 or 10, in which said at least one additional layer of material comprises:
- a first additional layer portion having a first additional thermal resistance T_{b}, and
- a second additional layer portion having a second additional thermal resistance T'_{b}, the first additional thermal resistance T_{b} and the second additional thermal resistance T'_{b} being different,
and in which said at least one additional temperature sensor comprises:
- at least one first additional temperature sensor disposed on an external surface of the first additional layer portion to measure a first additional peripheral temperature O_{b2},
- at least one second additional temperature sensor disposed on an external surface of the second additional layer portion to measure a second additional peripheral temperature Θ'_{b2},
the determination unit being configured to determine the conductor temperature Θ_{cond} further as a function of the first additional thermal resistance T_{b} and the second additional thermal resistance T'_{b} and the first additional peripheral temperature Θ_{b2} and the second additional peripheral temperature Θ'_{b2}.

12. The determination system (100) according to the preceding claim, in which said at least one first additional layer and said at least one second additional layer are disposed on different angular sectors around the electrical conductor.

13. The determination system (100) according to the preceding claim, in which said at least one temperature sensor (20) comprises:
- at least one first sensor disposed on the external surface (18) of said at least one layer of material, between said external surface (18) and the first additional layer portion, to measure a peripheral temperature Θ_{b1} at the level of the external surface of said at least one layer of material,
- at least one second sensor disposed on the external surface (18) of said at least one layer of material, between said external surface (18) and the second additional layer portion, to measure a peripheral temperature Θ'_{b1} at the level of the external surface of said at least one layer of material.

14. The determination system (100) according to the preceding claim, in which the determination unit is configured to determine the conductor temperature Θ_{cond} on the basis of the following equation: ${θ}_{conducteur} = \frac{\left({θ}_{b 1}{T}_{b}-θ{′}_{b 1}T{′}_{b}\right) \times \left(\left({θ}_{b 1}-{θ}_{b 2}\right)\right)}{{T}_{b} \left(θ{′}_{b 1}-θ{′}_{b 2}\right) - T {′}_{b} \left({θ}_{b 1}-{θ}_{b 2}\right)}$
T₁ being the thermal resistance of said at least one layer of material,
T_{b} being the first additional thermal resistance of the first additional layer of material, T'_{b} being the second additional thermal resistance of the second additional layer of material,
Θ_{b1} is the peripheral temperature measured by said at least one first temperature sensor,
Θ_{b2} is the additional peripheral temperature measured by said at least one first additional temperature sensor,
Θ'_{b1} is the peripheral temperature measured by said at least one second temperature sensor,
Θ'_{b2} is the additional peripheral temperature measured by said at least one second additional temperature sensor.

15. The determination system (100) according to any one of claims 11 to 14, in which the first and second additional layer portions are made of different materials.

16. The determination system (100) according to any one of claims 11 to 15, in which the first and second additional layer portions have different thicknesses perpendicular to a longitudinal axis along which the electrical cable (10) extends.
